# EUROPEAN PATENT SPECIFICATION

(11) **EP 2 150 599 B1**
(45) Date of publication and mention of the grant of the patent: **28.11.2012**
(21) Application number: 08769836.1
(22) Date of filing: 30.05.2008
(51) Int. Cl.: C09K 11/06, H05B 33/14, C07C 15/38, H01L 51/00, C07C 211/61

(54) **CHRYSENES FOR GREEN LUMINESCENT APPLICATIONS**
CHRYSENE FÜR GRÜN LUMINESZIERENDE ANWENDUNGEN
CHRYSÈNES UTILES POUR DES APPLICATIONS LUMINESCENTES DE COULEUR VERTE

(30) Priority: 01.06.2007 US 941351 P
(43) Date of publication of application: 10.02.2010
(73) Proprietor: E. I. Du Pont de Nemours and Company, Wilmington, DE 19898 (US)
(72) Inventor: ROSTOVTSEV, Vsevolod, Swarthmore, Pennsylvania 19081 (US); MERLO, Jeffrey, A., Wilmington, Delaware 19810 (US); HERRON, Norman, Newark, Delaware 19711 (US); DOBBS, Kerwin, D., Wilmington, Delaware 19803 (US)
(74) Representative: Hoffmann, Benjamin
(86) International application number: PCT/US2008/065187
(87) International publication number: WO 2008/150940

(56) References cited:
- EP-A- 1 561 794
- US-A1- 2006 152 146
- US-A1- 2006 210 830

## Description

### RELATED APPLICATION DATA

This application claims priority under 35 U.S.C. § 119(e) from U.S. Provisional Application No. 60/941,351 filed on June 1, 2007, which is incorporated by reference herein in its entirety.

### BACKGROUND

### Field of the Disclosure

This invention relates to electroluminescent chrysene compounds which have green emission. It also relates to electronic devices in which the active layer includes such a chrysene compound.

### Description of the Related Art

Organic electronic devices that emit light, such as light-emitting diodes that make up displays, are present in many different kinds of electronic equipment. In all such devices, an organic active layer is sandwiched between two electrical contact layers. At least one of the electrical contact layers is light-transmitting so that light can pass through the electrical contact layer. The organic active layer emits light through the light-transmitting electrical contact layer upon application of electricity across the electrical contact layers.

It is well known to use organic electroluminescent compounds as the active component in light-emitting diodes. Simple organic molecules such as anthracene, thiadiazole derivatives, and coumarin derivatives are known to show electroluminescence. Semiconductive conjugated polymers have also been used as electroluminescent components, as has been disclosed in, for example, U.S. Patent 5,247,190, U.S. Patent 5,408,109, and Published European Patent Application 443 861. Chrysene-based light-emitting compounds are disclosed in US 2006/0152146.

However, there is a continuing need for electroluminescent compounds, especially compounds that are green-emitting.

### SUMMARY

There is provided a compound having Formula I: 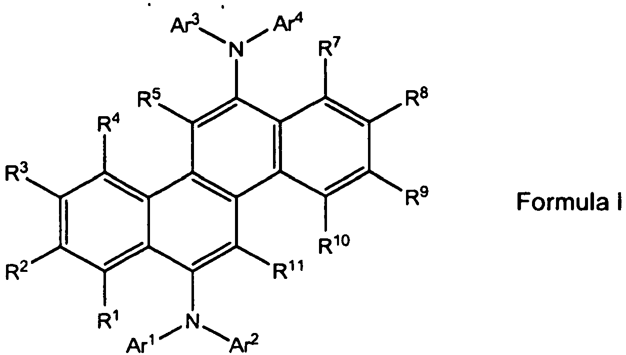 wherein:
Ar1 and Ar3 are the same or different and are aryl, and at least one of Ar1 and Ar3 has at least one alkyl substituent, with the proviso that there are no electron-withdrawing substituents;
Ar2 and Ar4 are the same or different and are aryl, with the proviso that there are no electron-withdrawing substituents;
R1, R2, and R4 are the same or different and are selected from the group consisting of H and an electron-withdrawing group;
R3 is an electron-withdrawing group;
R5 and R7 through R11 are the same or different and are selected from the group consisting of H and alkyl;
wherein the electron-withdrawing group is selected from group consisting of fluoro, perfluoroalkyl, cyano, nitro, -SO₂R, and combinations thereof, where R is alkyl or perfluoroalkyl, and
wherein said compound is capable of emitting green light.

There is also provided an electronic device comprising an active layer comprising the compound of Formula I.

### BRIEF DESCRIPTION OF THE DRAWINGS

Embodiments are illustrated in the accompanying figures to improve understanding of concepts as presented herein.

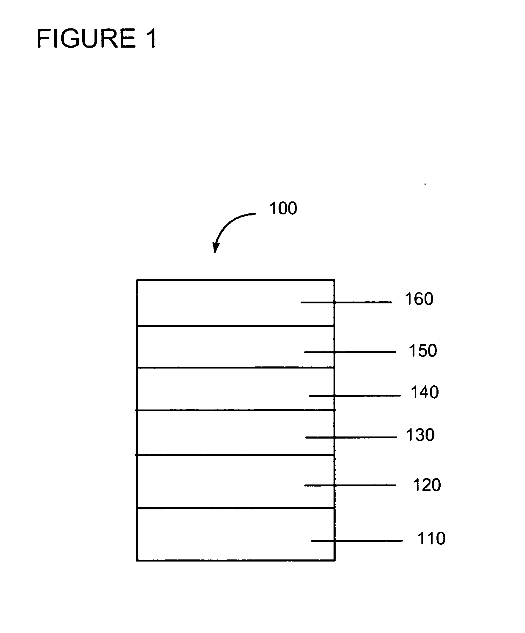

FIG. 1 includes an illustration of one example of an organic electronic device.

Skilled artisans appreciate that objects in the figures are illustrated for simplicity and clarity and have not necessarily been drawn to scale. For example, the dimensions of some of the objects in the figures may be exaggerated relative to other objects to help to improve understanding of embodiments.

### DETAILED DESCRIPTION

Many aspects and embodiments are disclosed herein and are exemplary and not limiting. After reading this specification, skilled artisans appreciate that other aspects and embodiments are possible without departing from the scope of the invention.

Other features and benefits of any one or more of the embodiments will be apparent from the following detailed description, and from the claims. The detailed description first addresses Definitions and Clarification of Terms followed by the Chrysene Compound, the Electronic Device, and finally Examples.

### 1. Definitions and Clarification of Terms

Before addressing details of embodiments described below, some terms are defined or clarified.

As used herein, the term "compound" is intended to mean an electrically uncharged substance made up of molecules that further consist of atoms, wherein the atoms cannot be separated by physical means. The phrase "adjacent to," when used to refer to layers in a device, does not necessarily mean that one layer is immediately next to another layer. On the other hand, the phrase "adjacent R groups," is used to refer to R groups that are next to each other in a chemical formula (i.e., R groups that are on atoms joined by a bond). The term "photoactive" refers to any material that exhibits electroluminescence and/or photosensitivity.

The term "electron withdrawing" as it refers to a substituent group is intended to mean a group which decreases the electron density of an aromatic ring.

The term "aryl" is intended to mean a group derived from an aromatic hydrocarbon having one point of attachment. The term includes groups which have a single ring and those which have multiple rings which can be joined by a single bond or fused together. The term is intended to include heteroaryls. The term "arylene" is intended to mean a group derived from an aromatic hydrocarbon having two points of attachment. In some embodiments, an aryl group has from 3-60 carbon atoms.

The term "alkyl" is intended to mean a group derived from an aliphatic hydrocarbon having one point of attachment, and includes a linear, a branched, or a cyclic group. The term is intended to include heteroalkyls. The term "alkylene" is intended to mean a group derived from an aliphatic hydrocarbon and having two or more points of attachment. In some embodiments, an alkyl group has from 1-20 carbon atoms.

The term "binaphthyl" is intended to mean a group having two naphthalene units joined by a single bond. In some embodiments, the binaphthyl group is 1,1-binaphthyl, which is attached at the 3-, 4-, or 5-position; in some embodiments, 1,2-binaphthyl, which is attached at the 3-, 4-, or 5-position on the 1-naphthyl moiety, or the 4- or 5-position on the 2-naphthyl moiety; and in some embodiments, 2,2-binaphthyl, which is attached at the 4- or 5-position.

The term "biphenyl" is intended to mean a group having two phenyl units joined by a single bond. The phenyl group can be attached at the 3-, 4-, or 5-position.

The prefix "fluoro" indicates that one or more hydrogen atoms have been replaced with a fluorine atom.

The term "green" refers to radiation that has an emission maximum at a wavelength in a range of approximately 500-600 nm.

The prefix "hetero" indicates that one or more carbon atoms have been replaced with a different atom. In some embodiments, the different atom is N, O, or S.

All groups may be unsubstituted or substituted, unless otherwise indicated. In some embodiments, the substituents are selected from the group consisting of alkyl, alkoxy, and aryl. The term "layer" is used interchangeably with the term "film" and refers to a coating covering a desired area. The term is not limited by size. The area can be as large as an entire device or as small as a specific functional area such as the actual visual display, or as small as a single sub-pixel. Layers and films can be formed by any conventional deposition technique, including vapor deposition, liquid deposition (continuous and discontinuous techniques), and thermal transfer. Continuous deposition techniques, include but are not limited to, spin coating, gravure coating, curtain coating, dip coating, slot-die coating, spray coating, and continuous nozzle coating. Discontinuous deposition techniques include, but are not limited to, ink jet printing, gravure printing, and screen printing.

The term "organic electronic device," or sometimes just "electronic device," is intended to mean a device including one or more organic semiconductor layers or materials.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. All publications, patent applications, patents, and other references mentioned herein are incorporated by reference in their entirety. In case of conflict, the present specification, including definitions, will control. In addition, the materials, methods, and examples are illustrative only and not intended to be limiting.

The IUPAC numbering system is used throughout, where the groups from the Periodic Table are numbered from left to right as 1-18 (CRC Handbook of Chemistry and Physics, 81st Edition, 2000).

### 2. Chrysene Compound

One aspect of the present invention is a compound of Formula I: 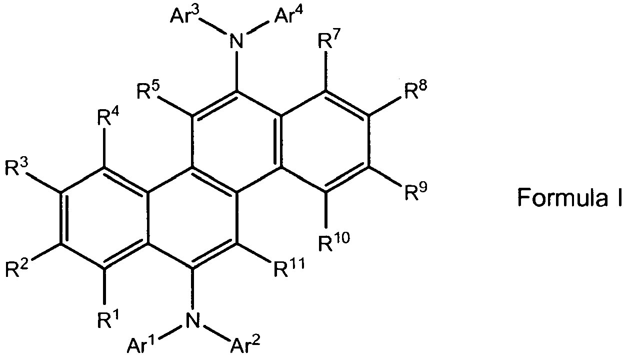 wherein:
Ar1 and Ar3 are the same or different and are aryl, and at least one of Ar1 and Ar3 has at least one alkyl substituent, with the proviso that there are no electron-withdrawing substituents;
Ar2 and Ar4 are the same or different and are aryl, with the proviso that there are no electron-withdrawing substituents;
R1, R2, and R4 are the same or different and are selected from the group consisting of H and an electron-withdrawing group;
R3 is an electron-withdrawing group;
R5 and R7 through R11 are the same or different and are selected from the group consisting of H and alkyl.
The compound is capable of green emission.

The electron-withdrawing group ("EWG") is selected from the group consisting of fluoro, perfluoroalkyl, cyano, nitro, -SO₂R, where R is alkyl or perfluoroalkyl, and combinations thereof. In some embodiments, the EWG is trifluoromethyl or cyano.

In some embodiments, both R1 and R3 are EWGs. In some embodiments, R2, R5, and R7 through R11 are H.

In some embodiments, Ar1 through Ar4 are independently selected from the group consisting of phenyl, biphenyl, naphthyl, and binaphthyl. In some embodiments, at least one of Ar1 through Ar4 is substituted with C1-20 alkyl. In some embodiments, the alkyl is branched.

In some embodiments, Ar1 and Ar3 are phenyl groups. In some embodiments, the phenyl groups have 1-5 alkyl substituents.

In some embodiments, at least one of Ar2 and Ar4 has at least one alkyl substituent. In some embodiments, the alkyl group has 1-8 carbon atoms. In some embodiments, both Ar2 and Ar4 are selected from the group consisting of phenyl and biphenyl.

In some embodiments, Ar1 and Ar3 are phenyl groups having at least one alkyl substituent and Ar2 and Ar4 are biphenyl groups having at least one alkyl substituent.

In some embodiments, the green chrysene compound has Formula E1:

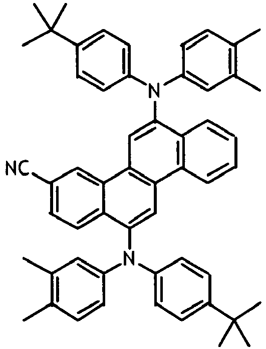

The new chrysenes can be prepared by known coupling and substitution reactions. An exemplary preparation is given in the Examples.

The chrysene compounds described herein can be formed into films using liquid deposition techniques. Thin films of these materials dispersed in a host matrix exhibit good to excellent photoluminescent properties and green emission.

### 3. Electronic Device

Organic electronic devices that may benefit from having one or more layers comprising the green luminescent materials described herein include, but are not limited to, (1) devices that convert electrical energy into radiation (e.g., a light-emitting diode, light emitting diode display, or diode laser), (2) devices that detect signals through electronics processes (e.g., photodetectors, photoconductive cells, photoresistors, photoswitches, phototransistors, phototubes, IR detectors), (3) devices that convert radiation into electrical energy, (e.g., a photovoltaic device or solar cell), and (4) devices that include one or more electronic components that include one or more organic semi-conductor layers (e.g., a transistor or diode).

One illustration of an organic electronic device structure is shown in Figure 1. The device 100 has a first electrical contact layer, an anode layer 110 and a second electrical contact layer, a cathode layer 160, and a photoactive layer 140 between them. Adjacent to the anode is a buffer layer 120. Adjacent to the buffer layer is a hole transport layer 130, comprising hole transport material. Adjacent to the cathode may be an electron transport layer 150, comprising an electron transport material. As an option, devices may use one or more additional hole injection or hole transport layers (not shown) next to the anode 110 and/or one or more additional electron injection or electron transport layers (not shown) next to the cathode 160.

Layers 120 through 150 are individually and collectively referred to as the active layers.

In one embodiment, the different layers have the following range of thicknesses: anode 110, 500-5000 Å, in one embodiment 1000-2000 Å; buffer layer 120, 50-2000 Å, in one embodiment 200-1000 Å; hole transport layer 130, 50-2000 Å, in one embodiment 200-1000 Å; photoactive layer 140, 10-2000 Å, in one embodiment 100-1000 Å; layer 150, 50-2000 Å, in one embodiment 100-1000 Å; cathode 160, 200-10000 Å, in one embodiment 300-5000 Å. The location of the electron-hole recombination zone in the device, and thus the emission spectrum of the device, can be affected by the relative thickness of each layer. The desired ratio of layer thicknesses will depend on the exact nature of the materials used.

Depending upon the application of the device 100, the photoactive layer 140 can be a light-emitting layer that is activated by an applied voltage (such as in a light-emitting diode or light-emitting electrochemical cell), or a layer of material that responds to radiant energy and generates a signal with or without an applied bias voltage (such as in a photodetector). Examples of photodetectors include photoconductive cells, photoresistors, photoswitches, phototransistors, and phototubes, and photovoltaic cells, as these terms are described in Markus, John, Electronics and Nucleonics Dictionary, 470 and 476 (McGraw-Hill, Inc. 1966).

### a. Photoactive layer

The chrysene compounds of Formula I are useful as photoactive materials in layer 140. The compounds can be used alone, or in combination with a host material.

In some embodiments, the host is a bis-condensed cyclic aromatic compound.

In some embodiments, the host is an anthracene derivative compound. In some embodiments the compound has the formula:

An-L-An

where:
An is an anthracene moiety;
L is a divalent connecting group.
In some embodiments of this formula, L is a single bond, -O-, -S-, - N(R)-, or an aromatic group. In some embodiments, An is a mono- or diphenylanthryl moiety.

In some embodiments, the host has the formula:

A-An-A

where:
An is an anthracene moiety;
A is the same or different at each occurrence and is an aromatic group.

In some embodiments, the A groups are attached at the 9- and 10-positions of the anthracene moiety. In some embodiments, A is selected from the group consisting naphthyl, naphthylphenylene, and naphthylnaphthylene. In some embodiments the compound is symmetrical and in some embodiments the compound is non-symmetrical.

In some embodiments, the host has the formula: 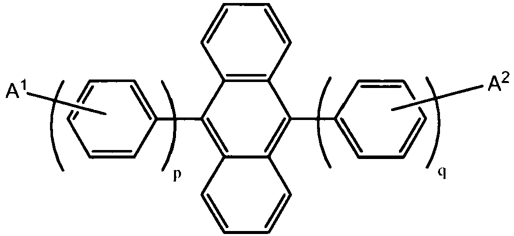 where:
A¹ and A² are the same or different at each occurrence and are selected from the group consisting of H, an aromatic group, and an alkenyl group, or A may represent one or more fused aromatic rings;
p and q are the same or different and are an integer from 1-3.
In some embodiments, the anthracene derivative is non-symmetrical. In some embodiments, p =2 and q = 1. In some embodiments, at least one of A¹ and A² is a naphthyl group.

In some embodiments, the host is selected from the group consisting of 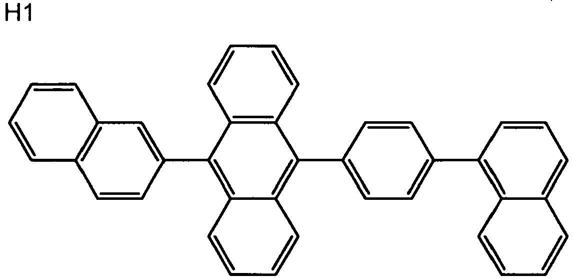 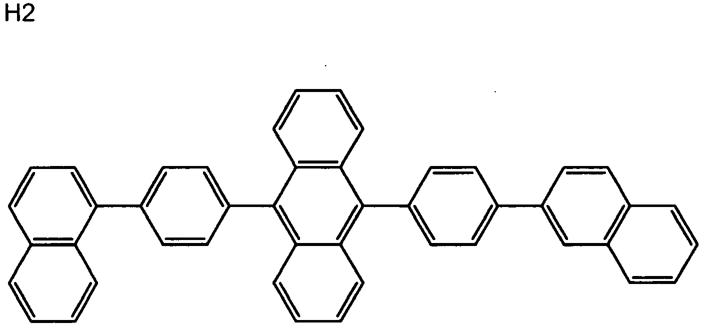 and combinations thereof.

The chrysene compounds of Formula I, in addition to being useful as emissive dopants in the photoactive layer, can also act as charge carrying hosts for other emissive dopants in the photoactive layer 140.

### b. Other Device Layers

The other layers in the device can be made of any materials that are known to be useful in such layers.

The anode 110, is an electrode that is particularly efficient for injecting positive charge carriers. It can be made of, for example, materials containing a metal, mixed metal, alloy, metal oxide or mixed-metal oxide, or it can be a conducting polymer, or mixtures thereof. Suitable metals include the Group 11 metals, the metals in Groups 4-6, and the Group 8-10 transition metals. If the anode is to be light-transmitting, mixed-metal oxides of Groups 12, 13 and 14 metals, such as indium-tin-oxide, are generally used. The anode 110 can also comprise an organic material such as polyaniline as described in "Flexible light-emitting diodes made from soluble conducting polymer," Nature vol. 357, pp 477-479 (11 June 1992). At least one of the anode and cathode is desirably at least partially transparent to allow the generated light to be observed.

The buffer layer 120 comprises buffer material and may have one or more functions in an organic electronic device, including but not limited to, planarization of the underlying layer, charge transport and/or charge injection properties, scavenging of impurities such as oxygen or metal ions, and other aspects to facilitate or to improve the performance of the organic electronic device. Buffer materials may be polymers, oligomers, or small molecules. They may be vapour deposited or deposited from liquids which may be in the form of solutions, dispersions, suspensions, emulsions, colloidal mixtures, or other compositions.

The buffer layer can be formed with polymeric materials, such as polyaniline (PANI) or polyethylenedioxythiophene (PEDOT), which are often doped with protonic acids. The protonic acids can be, for example, poly(styrenesulfonic acid), poly(2-acrylamido-2-methyl-1-propanesulfonic acid), and the like.

The buffer layer can comprise charge transfer compounds, and the like, such as copper phthalocyanine and the tetrathiafulvalene-tetracyanoquinodimethane system (TTF-TCNQ).

In some embodiments, the buffer layer comprises at least one electrically conductive polymer and at least one fluorinated acid polymer. Such materials have been described in, for example, published U.S. patent applications 2004-0102577, 2004-0127637, and 2005/205860

Examples of hole transport materials for layer 130 have been summarized for example, in Kirk-Othmer Encyclopedia of Chemical Technology, Fourth Edition, Vol. 18, p. 837-860, 1996, by Y. Wang. Both hole transporting molecules and polymers can be used. Commonly used hole transporting molecules are: N,N'-diphenyl-N,N'-bis(3-methylphenyl)-[1,1'-biphenyl]-4,4'-diamine (TPD), 1,1-bis[(di-4-tolylamino) phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis-(3-methylphenyl)-N,N,N',N'-2,5-phenylenediamine (PDA), a-phenyl-4-N,N-diphenylaminostyrene (TPS), p-(diethylamino)benzaldehyde diphenylhydrazone (DEH), triphenylamine (TPA), bis[4-(N,N-diethylamino)-2-methylphenyl](4-methylphenyl)methane (MPMP), 1-phenyl-3-[p-(diethylamino)styryl]-5-[p-(diethylamino)phenyl] pyrazoline (PPR or DEASP), 1,2-trans-bis(9H-carbazol-9-yl)cyclobutane (DCZB), N,N,N',N'-tetrakis(4-methylphenyl)-(1,1'-biphenyl)-4,4'-diamine (TTB), N,N'-bis(naphthalen-1-yl)-N,N'-bis-(phenyl)benzidine (α-NPB), and porphyrinic compounds, such as copper phthalocyanine. Commonly used hole transporting polymers are polyvinylcarbazole, (phenylmethyl)-polysilane, and polyaniline. It is also possible to obtain hole transporting polymers by doping hole transporting molecules such as those mentioned above into polymers such as polystyrene and polycarbonate. In some cases, triarylamine polymers are used, especially triarylamine-fluorene copolymers. In some cases, the polymers and copolymers are crosslinkable.

Examples of additional electron transport materials which can be used in layer 150 include metal chelated oxinoid compounds, such as tris(8-hydroxyquinolato)aluminum (Alq₃); bis(2-methyl-8-quinolinolato)(para-phenyl-phenolato)aluminum(III) (BAIQ); and azole compounds such as 2-(4-biphenylyl)-5-(4-t-butylphenyl)-1,3,4-oxadiazole (PBD) and 3-(4-biphenylyl)-4-phenyl-5-(4-t-butylphenyl)-1,2,4-triazole (TAZ), and 1,3,5-tri(phenyl-2-benzimidazole)benzene (TPBI); quinoxaline derivatives such as 2,3-bis(4-fluorophenyl)quinoxaline; phenanthroline derivatives such as 9,10-diphenylphenanthroline (DPA) and 2,9-dimethyl-4,7-diphenyl-1,10-phenanthroline (DDPA); and mixtures thereof. Layer 150 can function both to facilitate electron transport, and also serve as a buffer layer or confinement layer to prevent quenching of the exciton at layer interfaces. Preferably, this layer promotes electron mobility and reduces exciton quenching.

The cathode 160, is an electrode that is particularly efficient for injecting electrons or negative charge carriers. The cathode can be any metal or nonmetal having a lower work function than the anode. Materials for the cathode can be selected from alkali metals of Group 1 (e.g., Li, Cs), the Group 2 (alkaline earth) metals, the Group 12 metals, including the rare earth elements and lanthanides, and the actinides. Materials such as aluminum, indium, calcium, barium, samarium and magnesium, as well as combinations, can be used. Li-containing organometallic compounds, LiF, and Li₂O can also be deposited between the organic layer and the cathode layer to lower the operating voltage.

It is known to have other layers in organic electronic devices. For example, there can be a layer (not shown) between the anode 110 and buffer layer 120 to control the amount of positive charge injected and/or to provide band-gap matching of the layers, or to function as a protective layer. Layers that are known in the art can be used, such as copper phthalocyanine, silicon oxy-nitride, fluorocarbons, silanes, or an ultra-thin layer of a metal, such as Pt. Alternatively, some or all of anode layer 110, active layers 120, 130, 140, and 150, or cathode layer 160, can be surface-treated to increase charge carrier transport efficiency. The choice of materials for each of the component layers is preferably determined by balancing the positive and negative charges in the emitter layer to provide a device with high electroluminescence efficiency.

It is understood that each functional layer can be made up of more than one layer.

The device can be prepared by a variety of techniques, including sequential vapor deposition of the individual layers on a suitable substrate. Substrates such as glass, plastics, and metals can be used. Conventional vapor deposition techniques can be used, such as thermal evaporation, chemical vapor deposition, and the like. Alternatively, the organic layers can be applied from solutions or dispersions in suitable solvents, using conventional coating or printing techniques, including but not limited to spin-coating, dip-coating, roll-to-roll techniques, ink-jet printing, screenprinting, gravure printing and the like. In general, the different layers will have the following range of thicknesses: anode 110, 500-5000Å, preferably 1000-2000Å; hole transport layer

The present invention also relates to an electronic device comprising at least one active layer positioned between two electrical contact layers, wherein the at least one layer of the device includes the chrysene compound of Formula 1. Devices frequently have additional hole transport and electron transport layers.

To achieve a high efficiency LED, the HOMO (highest occupied molecular orbital) of the hole transport material desirably aligns with the work function of the anode, and the LUMO (lowest un-occupied molecular orbital) of the electron transport material desirably aligns with the work function of the cathode. Chemical compatibility and sublimation temperature of the materials are also important considerations in selecting the electron and hole transport materials.

It is understood that the efficiency of devices made with the chrysene compounds described herein, can be further improved by optimizing the other layers in the device. For example, more efficient cathodes such as Ca, Ba or LiF can be used. Shaped substrates and novel hole transport materials that result in a reduction in operating voltage or increase quantum efficiency are also applicable. Additional layers can also be added to tailor the energy levels of the various layers and facilitate electroluminescence.

The chrysene compounds of the invention often are fluorescent and photoluminescent and can be useful in applications other than OLEDs, such as oxygen sensitive indicators and as fluorescent indicators in bioassays.

### EXAMPLES

The following examples illustrate certain features and advantages of the present invention. They are intended to be illustrative of the invention, but not limiting. All percentages are by weight, unless otherwise indicated.

### Example 1

This example illustrates the preparation of Compound E1, 6,12-bis((4-*tert*-butylphenyl)(3,4-dimethylphenyl)amino)chrysene-3-carbonitrile

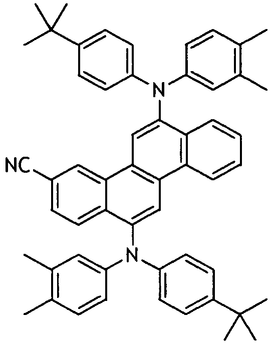

### a. Preparation of 4-(2-(naphthalen-1-yl)vinyl)benzonitrile

An oven-dried 500 ml three-neck flask was purged with nitrogen and charged with (naphthalen-1-ylmethyl)triphenylphosphonium (25 g, 57 mmol) and dry THF (250 ml). Sodium hydride (1.4 g, 58 mmol) was added and reaction mixture was left to stir overnight at room temperature. Solution was red-orange in the morning. 4-Cyanobenzaldehyde (6.79 g, 52 mmol) was added next day (16 h later), bleaching the orange-red color. Reaction mixture was stirred for 5 hours. THF was removed under reduced pressure. White residue was suspended in hexane and filtered to get rid of Ph₃PO. Filter cake was washed with hexane (700 ml). Filtrates were combined and hexanes were removed in vacuo. Crude product was purified by column chromoatography in 100% CH₂Cl₂. The desired product is a mixture of cis- and trans-isomers. Yield 6.1 g (46%). The structure was confirmed by ¹H NMR spectroscopy.

### b. Preparation of 3-cyanochrysene.

A mixture of isomeric 4-(2-(naphthalen-1-yl)vinyl)benzonitriles (1 g, 3.92 mmol) was dissolved in 10 ml of dry toluene and transferred into a 1 L photochemical vessel, equipped with a nitrogen inlet and a stirbar. Next, dry toluene (1 L) was added by cannula, followed by iodine (1 g, 3.94 mmol) and propylene oxide (50 ml, 714 mmol, 183 equiv.). Two condensers were attached on top of the photochemical vessel. The halogen lamp (Hanovia, 450 W) was turned on. Reaction was stopped by turning off the lamp when no more iodine was left in the reaction mixture, as evidenced by the disappearance of its color. Toluene and excess propylene oxide were removed under reduced pressure to give ca. 0.9 g of yellow solid. The solid was precipitated from CH₂Cl₂ with hexane at low temperature, affording white needles (0.75 g , 75.5 %). The structure of this material was confirmed by ¹H NMR spectroscopy.

### c. Preparation of 6,12-dibromo-3-cyanochrysene

3-Cyanochrysene (1 g, 3.94 mmol) was placed into a 100 ml roundbottom flaskand suspended in 30 ml of (MeO)₃PO. Bromine (1.59 g, 10 mmol) was added next. Condenser was attached to the flask, which was brought to 110 °C and stirred for 1 hour. Reaction mixture was then cooled to room temperature and poured into 250 ml of water. The resulting precipitate was filtered again and washed with 100 ml of diethyl ether, giving 1.43 g (88.3 %) of the yellow product. The identity of the product was confirmed by ¹H NMR spectroscopy.

### d. Preparation of E1

In a drybox, 6,12-dibromo-3-cyanochrysene (1.42 g, 3.45 mmol) and N-(4-*tert*-butylphenyl)-3,4-dimethylaniline (1.79 g, 7.08 mmol) were combined in a thick-walled glass tube and dissolved in 15 ml of toluene. Tris(*tert*-butyl)phosphine (0.056 g, 0.28 mmol) and tris(dibenzylideneacetone)dipalladium(0) (0.127 g, 0.14 mmol) were added next, followed by sodium *tert*-butoxide (0.797 g, 8.29 mmol) and 10 ml of dry toluene. Glass tube was sealed, brought out of the box and placed into a 100 °C oil bath for 24 hours. Reaction mixture was cooled to room temperature, diluted with 100 ml of dichloromethane and filtered through a plug of celite and silica. The plug was washed with additional 200 ml of dichloromethane. Filtrates were combined and volatiles were removed under reduced pressure to give dark yellow solid. Further purification was done by column chromatography on silica using 15% dichloromethane in hexane. The crude product was purified again on a silica column again (15% CH₂Cl₂ in hexane) and then on a florosil column (30% CH₂Cl₂ in hexane). The resulting yellow powder (0.5 g or 20%) was 97.3 % pure by LC. The structure was confirmed by ¹H NMR and NOESY spectroscopy. ¹H NMR (CD₂Cl₂): δ 1.19 (s, 9H), 1.20 (s, 9H), 2.04 (s, 3H), 2.05 (s, 3H) 2.109 (s, 3H), 2.112 (s, 3H), 6.75 (app dt, 2H, J = 2.1 Hz, J = 5.9 Hz), 6.82-6.93 (m, 8H), 7.14 (d, 4H, J = 8.9 Hz), 7.42 (br t, 1H, J = 7.2 Hz), 7.47-7.54 (m, 2H), 8.07 (d, 1H, J = 8.2 Hz), 8.13 (d, 1H, J = 8.6 Hz), 8.37 (s, 1H), 8.44 (d, 1H, J = 8.6Hz), 8.58 (s, 1H), 8.81 (s, 1H).

### Example 2

This example demonstrates the fabrication and performance of a device having green emission. The following materials were used:
Indium Tin Oxide (ITO): 100 nm
buffer layer = Buffer 1 (25 nm), which is an aqueous dispersion of polypyrrole and a polymeric fluorinated sulfonic acid. The material was prepared using a procedure similar to that described in Example 1 of published U.S. patent application no. 2005/0205860.
hole transport layer = polymer P1 (20 nm)
photoactive layer = 13:1 host H2:dopant E1 (48 nm)
electron transport layer = Tetrakis-(8-hydroxyquinoline) zirconium (ZrQ) (20 nm)
cathode = LiF/Al (0.5/100 nm)

OLED devices were fabricated by a combination of solution processing and thermal evaporation techniques. Patterned indium tin oxide (ITO) coated glass substrates from Thin Film Devices, Inc were used. These ITO substrates are based on Corning 1737 glass coated with ITO having a sheet resistance of 30 ohms/square and 80% light transmission. The patterned ITO substrates were cleaned ultrasonically in aqueous detergent solution and rinsed with distilled water. The patterned ITO was subsequently cleaned ultrasonically in acetone, rinsed with isopropanol, and dried in a stream of nitrogen.

Immediately before device fabrication the cleaned, patterned ITO substrates were treated with UV ozone for 10 minutes. Immediately after cooling, an aqueous dispersion of Buffer 1 was spin-coated over the ITO surface and heated to remove solvent. After cooling, the substrates were then spin-coated with a solution of a hole transport material, and then heated to remove solvent. After cooling the substrates were spin-coated with the emissive layer solution, and heated to remove solvent. The substrates were masked and placed in a vacuum chamber. A ZrQ layer was deposited by thermal evaporation, followed by a layer of LiF. Masks were then changed in vacuo and a layer of Al was deposited by thermal evaporation. The chamber was vented, and the devices were encapsulated using a glass lid, dessicant, and UV curable epoxy.

The OLED samples were characterized by measuring their (1) current-voltage (I-V) curves, (2) electroluminescence radiance versus voltage, and (3) electroluminescence spectra versus voltage. All three measurements were performed at the same time and controlled by a computer. The current efficiency of the device at a certain voltage is determined by dividing the electroluminescence radiance of the LED by the current density needed to run the device. The unit is a cd/A. The power efficiency is the current efficiency divided by the operating voltage. The unit is Im/W. The results are given in Table 1.

**TABLE 1**

| **Example** | **CE [cd/A]** | **Voltage (V)** | **EL peak [nm]** | **CIE [x]** | **CIE [y]** | **Lum. ½ Life [h]** |
|---|---|---|---|---|---|---|
| 2 | 15.4 | 4.3 | 512 | 0.24 | 0.61 | 140,000 |

| | | | | | | |
|---|---|---|---|---|---|---|
| * All data @ 1000 nits, CE = current efficiency | | | | | | |

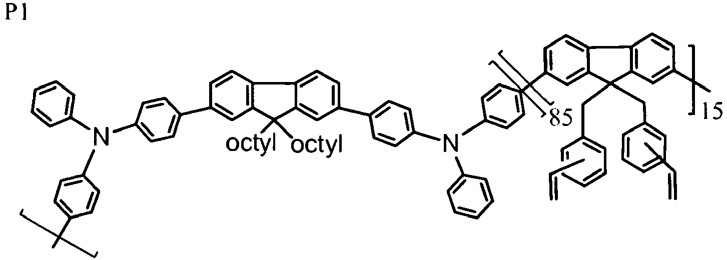

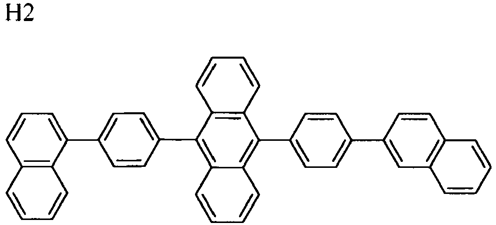

Note that not all of the activities described above in the general description or the examples are required, that a portion of a specific activity may not be required, and that one or more further activities may be performed in addition to those described. Still further, the order in which activities are listed are not necessarily the order in which they are performed.

In the foregoing specification, the concepts have been described with reference to specific embodiments. However, one of ordinary skill in the art appreciates that various modifications and changes can be made without departing from the scope of the invention as set forth in the claims below. Accordingly, the specification and figures are to be regarded in an illustrative rather than a restrictive sense, and all such modifications are intended to be included within the scope of invention.

Benefits, other advantages, and solutions to problems have been described above with regard to specific embodiments. However, the benefits, advantages, solutions to problems, and any feature(s) that may cause any benefit, advantage, or solution to occur or become more pronounced are not to be construed as a critical, required, or essential feature of any or all the claims.

The use of numerical values in the various ranges specified herein is stated as approximations as though the minimum and maximum values within the stated ranges were both being preceded by the word "about." In this manner slight variations above and below the stated ranges can be used to achieve substantially the same results as values within the ranges. Also, the disclosure of these ranges is intended as a continuous range including every value between the minimum and maximum average values including fractional values that can result when some of components of one value are mixed with those of different value. Moreover, when broader and narrower ranges are disclosed, it is within the contemplation of this invention to match a minimum value from one range with a maximum value from another range and vice versa.

It is to be appreciated that certain features are, for clarity, described herein in the context of separate embodiments, may also be provided in combination in a single embodiment. Conversely, various features that are, for brevity, described in the context of a single embodiment, may also be provided separately or in any subcombination.

## Claims

1. A compound having Formula I: 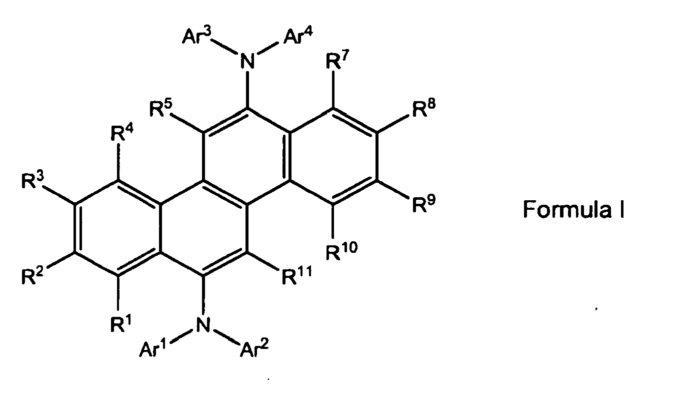 wherein:
Ar1 and Ar3 are the same or different and are aryl, and at least one of Ar1 and Ar3 has at least one alkyl substituent, with the proviso that there are no electron-withdrawing substituents;
Ar2 and Ar4 are the same or different and are aryl, with the proviso that there are no electron-withdrawing substituents;
R1, R2, and R4 are the same or different and are selected from the group consisting of H and an electron-withdrawing group;
R3 is an electron-withdrawing group; R
R5 and R7 through R11 are the same or different and are selected from the group consisting of H and alkyl;
wherein the electron-withdrawing group is selected from group consisting of fluoro, perfluoroalkyl, cyano, nitro, -SO₂R, and combinations thereof, where R is alkyl or perfluoroalkyl, and
wherein said compound is capable of emitting green light.

2. The compound of Claim 1, wherein the electron-withdrawing group is selected from trifluoromethyl and cyano.

3. The compound of Claim 1, wherein R1 is an electron-withdrawing group.

4. The compound of Claim 3, wherein R2, R5, and R7 through R11 are H.

5. The compound of Claim 1, wherein Ar1 through Ar4 are independently selected from the group consisting of phenyl, biphenyl, naphthyl, and binaphthyl.

6. The compound of Claim 1, wherein Ar3 has at least one alkyl substituent.

7. The compound of Claim 1, wherein Ar2 and Ar4 each have at least one alkyl substituent.

8. The compound of Claim 1, wherein Ar1 and Ar3 are phenyl, and Ar2 and Ar4 are biphenyl.

9. An organic electronic device comprising a first electrical contact layer, a second electrical contact layer, and at least one active layer therebetween, wherein the active layer comprises a compound having Formula I: 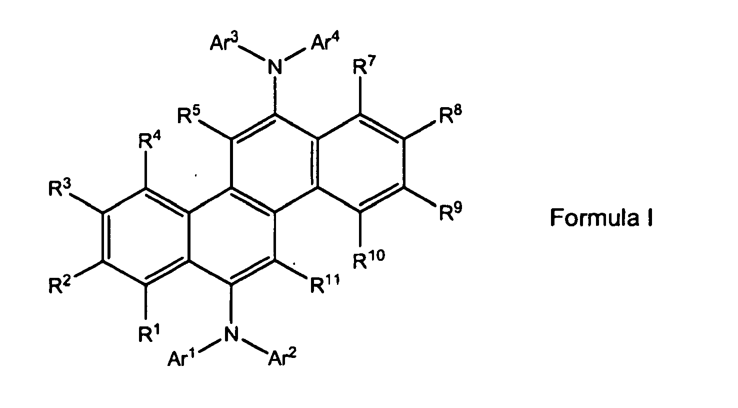 wherein:
Ar1 and Ar3 are the same or different and are aryl, and at least one of Ar1 and Ar3 has at least one alkyl substituent, with the proviso that there are no electron-withdrawing substituents;
Ar2 and Ar4 are the same or different and are aryl, with the proviso that there are no electron-withdrawing substituents;
R1, R2, and R4 are the same or different and are selected from the group consisting of H and an electron-withdrawing group;
R3 is an electron-withdrawing group;
R5 and R7 through R11 are the same or different and are selected from the group consisting of H and alkyl;
wherein the electron-withdrawing group is selected from group consisting of fluoro, perfluoroalkyl, cyano, nitro, -SO₂R, and combinations thereof, where R is alkyl or perfluoroalkyl. and
wherein said compound is capable of emitting green light.

10. The device of Claim 9 wherein the active layer further comprises a host material.

11. An active layer comprising a compound as in any one of Claims 1-8.

12. The active layer of Claim 11 further comprising a host material having a formula selected from the group consisting of,
An-L-An
where:
An is an anthracene moiety;
L is a divalent connecting group; and
A-An-A
where:
An is an anthracene moiety;
A is the same or different at each occurrence and is an aromatic group.

13. The active layer of Claim 12 wherein the host has the formula, 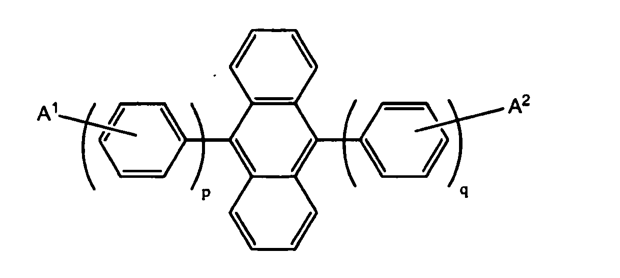 where:
A¹ and A² are the same or different at each occurrence and are selected from the group consisting of H, an aromatic group, and an alkenyl group, or A may represent one or more fused aromatic rings;
p and q are the same or different and are an integer from 1-3.

14. The active layer of Claim 12 wherein the host is selected from the group consisting of 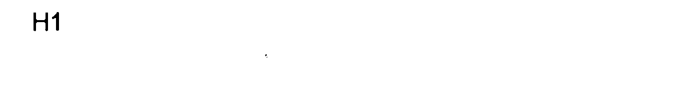 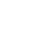 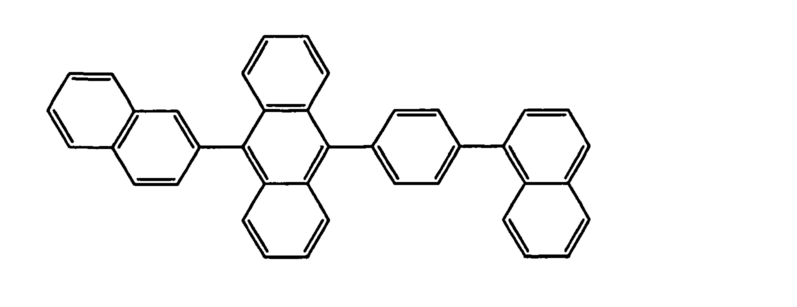 and 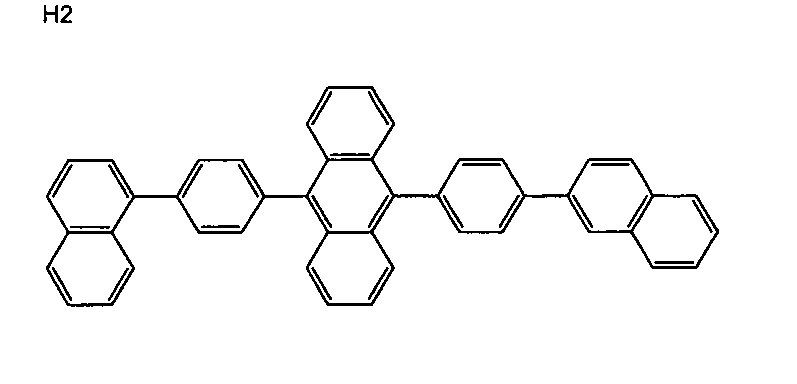 and combinations thereof.

## Patentansprüche

1. Verbindung, die die Formel I: 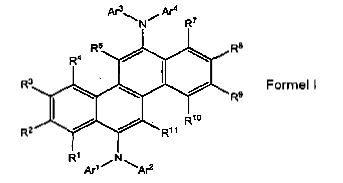 aufweist, wobei
Ar1 und Ar3 gleich oder verschieden und Aryl sind und mindestens eines von Ar1 und Ar3 mindestens einen Alkylsubstituenten aufweist, mit der Maßgabe, dass keine elektronenziehenden Substituenten vorliegen;
Ar2 und Ar4 gleich oder verschieden und Aryl sind, mit der Maßgabe, dass keine elektronenziehenden Substituenten vorliegen;
R1, R2 und R4 gleich oder verschieden und aus der Gruppe ausgewählt sind bestehend aus H und einer elektronenziehenden Gruppe;
R3 eine elektronenziehende Gruppe ist;
R5 und R7 bis R11 gleich oder verschieden und aus der Gruppe bestehend aus H und Alkyl ausgewählt sind;
wobei die elektronenziehende Gruppe aus der Gruppe ausgewählt ist bestehend aus
Fluor, Perfluoralkyl, Cyano, Nitro, -SO₂R und Kombinationen davon,
wobei R ein Alkyl oder Perfluoralkyl ist und
wobei die Verbindung in der Lage ist, grünes Licht auszustrahlen.

2. Verbindung nach Anspruch 1, wobei die elektronenziehende Gruppe von Trifluormethyl und Cyano ausgewählt ist.

3. Verbindung nach Anspruch 1, wobei R1 eine elektronenziehende Gruppe ist.

4. Verbindung nach Anspruch 3, wobei R2, R5 und R7 bis R11 H sind.

5. Verbindung nach Anspruch 1, wobei Ar1 bis Ar4 unabhängig aus der Gruppe ausgewählt sind bestehend aus Phenyl, Biphenyl, Naphthyl und Binaphthyl.

6. Verbindung nach Anspruch 1, wobei Ar3 mindestens einen Alkylsubstituenten aufweist.

7. Verbindung nach Anspruch 1, wobei Ar2 und Ar4 jeweils mindestens einen Alkylsubstituenten aufweisen.

8. Verbindung nach Anspruch 1, wobei Ar1 und Ar3 Phenyl sind und Ar2 und Ar4 Biphenyl sind.

9. Organische elektronische Vorrichtung umfassend eine erste elektrische Kontaktschicht, eine zweite elektrische Kontaktschicht und mindestens eine aktive Schicht dazwischen, wobei die aktive Schicht eine Verbindung umfasst, die die Formel I: 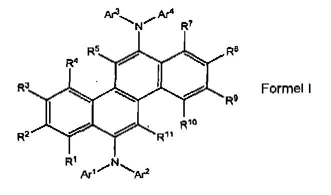 aufweist, wobei:
Ar1 und Ar3 gleich oder verschieden und Aryl sind und mindestens eines von Ar1 und Ar3 mindestens einen Alkylsubstituenten aufweist, mit der Maßgabe, dass keine elektronenziehenden Substituenten vorliegen;
Ar2 und Ar4 gleich oder verschieden und Aryl sind, mit der Maßgabe, dass keine elektronenziehenden Substituenten vorliegen;
R1, R2 und R4 gleich oder verschieden und aus der Gruppe ausgewählt sind bestehend aus H und einer elektronenziehenden Gruppe;
R3 eine elektronenziehende Gruppe ist;
R5 und R7 bis R11 gleich oder verschieden und aus der Gruppe bestehend aus H und Alkyl ausgewählt sind;
wobei die elektronenziehende Gruppe aus der Gruppe ausgewählt ist bestehend aus Fluor, Perfluoralkyl, Cyano, Nitro, -SO₂R und Kombinationen davon,
wobei R ein Alkyl oder Perfluoralkyl ist und
wobei die Verbindung in der Lage ist, grünes Licht auszustrahlen.

10. Vorrichtung nach Anspruch 9, wobei die aktive Schicht des Weiteren ein Wirtsmaterial umfasst.

11. Aktive Schicht umfassend eine Verbindung wie in einem der Ansprüche 1 bis 8.

12. Aktive Schicht nach Anspruch 11, des Weiteren ein Wirtsmaterial umfassend, das eine Formel aufweist, die aus der Gruppe ausgewählt ist bestehend aus
An-L-An
wobei:
An ein Anthracenanteil ist;
L eine zweiwertige Verbindungsgruppe ist; und
A-An-A
wobei:
An ein Anthracenanteil ist;
A bei jedem Vorkommen gleich oder verschieden und eine aromatische Gruppe ist.

13. Aktive Schicht nach Anspruch 12, wobei der Wirt die Formel 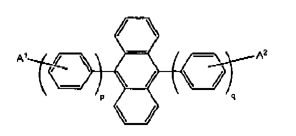 aufweist, wobei:
A¹ und A² bei jedem Vorkommen gleich oder verschieden sind und aus der Gruppe ausgewählt sind bestehend aus H, einer aromatischen Gruppe und einer Alkenylgruppe oder A einen oder mehrere anellierte aromatische Ringe darstellen kann;
p und q gleich oder verschieden und ganze Zahlen von 1 bis 3 sind.

14. Aktive Schicht nach Anspruch 12, wobei der Wirt aus der Gruppe ausgewählt ist bestehend aus 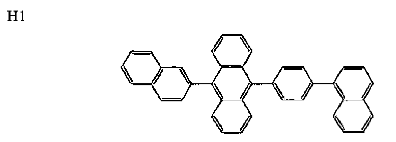 und 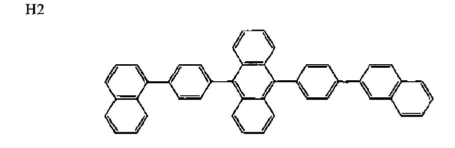 und Kombinationen davon.

## Revendications

1. Composé de formule I: 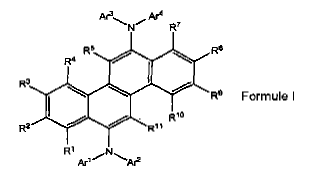 dans laquelle:
Ar1 et Ar3 sont identiques ou différents et sont un groupe aryle, et au moins l'un entre Ar1 et Ar3 a au moins un substituant alkyle, à condition qu'ils ne soient pas des substituants de retrait d'électrons;
Ar2 et Ar4 sont identiques ou différents et sont un groupe aryle, à condition qu'ils ne soient pas des substituants de retrait d'électrons;
R1, R2, et R4 sont identiques ou différents et sont sélectionnés parmi le groupe constitué d'un atome H et d'un groupe de retrait d'électrons;
R3 est un groupe de retrait d'électrons;
R5 et R7 jusqu'à R11 sont identiques ou différents et sont sélectionnés parmi le groupe constitué d'un atome H et d'un groupe alkyle;
dans laquelle le groupe de retrait d'électrons est sélectionné parmi le groupe constitué de
un groupe fluoro, perfluoroalkyle, cyano, nitro, -SO₂R, et leurs combinaisons,
où R est un groupe alkyle ou perfluoroalkyle, et
dans lequel ledit composé est capable d'émettre de la lumière verte.

2. Composé selon la revendication 1, dans lequel le groupe de retrait d'électrons est sélectionné parmi le groupe trifluorométhyle et cyano.

3. Composé selon la revendication 1, dans lequel R1 est un groupe de retrait d'électrons.

4. Composé selon la revendication 3, dans lequel R2, R5, et R7 jusqu'à R11 sont un atome H.

5. Composé selon la revendication 1, dans lequel Ar1 jusqu'à Ar4 sont indépendamment choisis parmi le groupe constitué du groupe phényle, biphényle, naphtyle, et binaphtyle.

6. Composé selon la revendication 1, dans lequel Ar3 a au moins un substituant alkyle.

7. Composé selon la revendication 1, dans lequel Ar2 et Ar4 ont chacun au moins un substituant alkyle.

8. Composé selon la revendication 1, dans lequel Ar1 et Ar3 sont un groupe phényle, et Ar2 et Ar4 sont un groupe biphényle.

9. Dispositif électronique organique comprenant une première couche de contact électrique, une seconde couche de contact électrique et au moins une couche active entre les deux, dans lequel la couche active comprend un composé ayant la formule I: 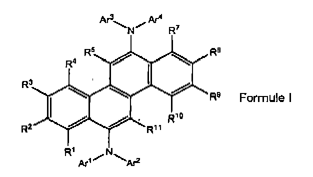 dans laquelle:
Ar1 et Ar3 sont identiques ou différents et sont un groupe aryle, et au moins l'un de Ar1 et Ar3 a au moins un substituant alkyle, à condition qu'il n'existe pas de substituants de retrait d'électrons;
Ar2 et Ar4 sont identiques ou différents et sont un groupe aryle, à condition qu'il n'existe pas de substituants de retrait d'électrons;
R1, R2, et R4 sont identiques ou différents et sont sélectionnés parmi le groupe constitué d'un atome H et d'un groupe de retrait d'électrons;
R3 est un groupe de retrait d'électrons;
R5 et R7 jusqu'à R11 sont identiques ou différents et sont sélectionnés parmi le groupe constitué d'un atome H et d'un groupe alkyle;
où le groupe de retrait d'électrons est sélectionné parmi le groupe constitué du groupe fluoro, perfluoroalkyle, cyano, nitro, -SO₂R, et leurs combinaisons,
où R est un groupe alkyle ou perfluoroalkyle, et
où ledit composé est capable d'émettre de la lumière verte.

10. Dispositif selon la revendication 9, dans lequel la couche active comprend en outre un matériau hôte.

11. Couche active comprenant un composé selon l'une quelconque des revendications 1 à 8.

12. Couche active selon la revendication 11, comprenant en outre un matériau hôte ayant une formule sélectionnée parmi le groupe constitué de,
An-L-An
où:
An est une fraction anthracène;
L est un groupe de liaison divalent; et
A-An-A
où:
An est une fraction anthracène;
A est identique ou différent à chaque occurrence et est un groupe aromatique.

13. Couche active selon la revendication 12, dans laquelle l'hôte a la formule, 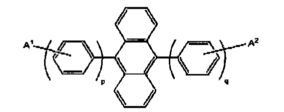 où:
A1 et A2 sont identiques ou différents à chaque occurrence et sont sélectionnés parmi le groupe constitué d'un atome H, d'un groupe aromatique, et d'un groupe alcényle, ou A peut représenter un ou plusieurs cycle(s) aromatique(s) condensé(s);
p et q sont identiques ou différents et sont un nombre entier d'une valeur de 1 à 3.

14. Couche active selon la revendication 12, dans laquelle l'hôte est sélectionné parmi le groupe constitué de 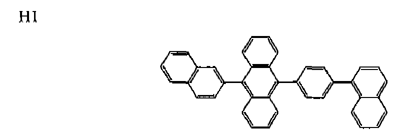 et 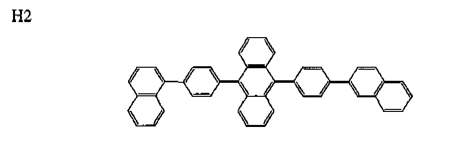 et leurs combinaisons.
